# EUROPEAN PATENT SPECIFICATION

(11) **EP 3 050 491 B1**
(45) Date of publication and mention of the grant of the patent: **10.10.2018**
(21) Application number: 14846840.8
(22) Date of filing: 10.04.2014
(51) Int. Cl.: A61B 1/04, A61B 1/00, G02B 23/24, G02B 23/26, H04N 5/225, H05K 1/02, A61B 1/05, A61B 1/12

(54) **ENDOSCOPE DEVICE**
ENDOSKOPVORRICHTUNG
DISPOSITIF D'ENDOSCOPE

(30) Priority: 26.09.2013 JP 2013200645
(43) Date of publication of application: 03.08.2016
(73) Proprietor: Olympus Corporation, Tokyo 192-8507 (JP)
(72) Inventor: ICHIMURA, Hironobu, Hachioji-shi, Tokyo 192-8507 (JP); TAKAHASHI, Tomohisa, Hachioji-shi, Tokyo 192-8507 (JP); FUJIMORI, Noriyuki, Hachioji-shi, Tokyo 192-8507 (JP); IGARASHI, Takatoshi, Hachioji-shi, Tokyo 192-8507 (JP)
(74) Representative: Gunzelmann, Rainer
(86) International application number: PCT/JP2014/060383
(87) International publication number: WO 2015/045456

(56) References cited:
- JP-A- 2010 279 527
- JP-A- 2010 279 527
- JP-A- 2011 050 497
- JP-A- 2011 188 375
- JP-A- 2011 188 375
- JP-A- 2011 217 887
- JP-A- 2011 217 887
- US-A1- 2008 023 784

## Description

### Field

The present invention relates to an endoscope apparatus in which an imaging unit which captures the inside of a subject is disposed on the distal end of an insertion part inserted into the subject.

### Background

Conventionally, in the medical field and the industrial field, endoscope apparatuses have been widely used for various inspections. Among these endoscope apparatuses, a medical endoscope apparatus is capable of acquiring an in-vivo image inside the body cavity without making an incision on a subject such as a patient by inserting an elongated and flexible insertion part having a solid-state imaging element disposed on the distal end thereof into the body cavity of the subject and further capable of performing a therapeutic treatment by allowing a treatment tool to project from the distal end of the insertion part as needed, and thus widely used.

In such an endoscope apparatus, it is required to dissipate heat generated by driving the solid-state imaging element to ensure the electrical characteristics of the solid-state imaging element. For example, Patent Literature 1 proposes arranging a heat dissipation member having a high thermal conductivity in contact with the solid-state imaging element.

Document JP 2010 279527 A discloses an endoscope comprising a solid-state image sensing device, wherein a proximal side of the solid-state imaging sensing device is connected to a distal side of a heat dissipation member. The proximal side of the heat dissipation member is connected to a distal side of a driving circuit board, wherein the proximal side of the driving circuit board is connected to wires.

Document US 2008/023784 A1 discloses a solid-state imaging device chip, wherein a side face of the solid-state imaging device chip is connected to one side face of a base film. The other side face opposite the one side face of the base film is connected to a side face of a wiring pattern, wherein the other side face of the wiring pattern is connected to a cover layer. The wiring pattern is connected via a connection and a wire to an electrode pad connected to the solid-state device chip. Additionally, a part of the wiring pattern connected to the connection has an increased with compared to the other part of the wiring pattern.

Document JP 2011 217887 A relates to an imaging apparatus discloses a signal cable, wherein a plurality of conductive wires extends from the signal cable and is connected to respective thermal portions provided on a wiring board. Another side face of the wiring board is connected to a front surface of an image pickup element.

Document JP 2011 188375 A relates to an imaging device and discloses a plurality of signal cables, which are inserted into respective connection holes of a wiring board. The wiring board is connected via connection terminals to a CCD.

### Citation List

### Patent Literature

Patent Literature 1: Japanese Laid-open Patent Publication No. 2002-291693 Summary

### Technical Problem

In the technique described in Patent Literature 1, the heat dissipation member which has no relation to an electric signal is arranged near the solid-state imaging element. This configuration makes it difficult to downsize the distal end of the insertion part of the endoscope on which the solid-state imaging element is mounted.

The present invention has been made in view of the above, and an object thereof is to provide an endoscope apparatus that efficiently dissipates heat generated by a solid-state imaging element and, at the same time, achieves downsizing of an endoscope.

### Solution to Problem

To solve the above-described problem and achieve the object, an endoscope apparatus according independent claim 1 is disclosed. The endoscope apparatus includes: a solid-state imaging element including a light receiving surface on a front face thereof; a circuit board arranged on a rear face side of the solid-state imaging element, the circuit board including a wiring pattern a part of which is exposed on a distal end side of the circuit board, the distal end side facing the solid-state imaging element; a first heat dissipation member arranged between the solid-state imaging element and the exposed part of the wiring pattern; and a cable electrically connected to the wiring pattern.

Moreover, in the above-described endoscope apparatus according to the present invention, the circuit board includes: a laminated circuit board including a plurality of conductor layers laminated in a direction parallel to the light receiving surface; and a soft circuit board configured to connect the solid-state imaging element electrically to the laminated circuit board, and the part of the wiring pattern is exposed on an end face of the laminated circuit board, the end face facing the rear face
of the solid-state imaging element.

Moreover, the above-described endoscope apparatus according to the present invention further includes an insulative second heat dissipation member configured to cover the cable on a proximal end side of the circuit board.

Moreover, in the above-described endoscope apparatus according to the present invention, a part of a wiring pattern electrically connected to the wiring pattern exposed on the distal end side is exposed on the proximal end side of the circuit board.

Moreover, in the above-described endoscope apparatus according to the present invention, the first heat dissipation member is an adhesive that fixes the solid-state imaging element and the laminated circuit board to each other.

Moreover, in the above-described endoscope apparatus according to the present invention, the first heat dissipation member is an insulative member.

Moreover, in the above-described endoscope apparatus according to the present invention, the first heat dissipation member is an electrically conductive member, and the wiring pattern is a pattern for ground or power source.

### Advantageous Effects of Invention

According to the present invention, there is provided an endoscope apparatus that efficiently dissipates heat generated by a solid-state imaging element and, at the same time, achieves downsizing of an endoscope.

### Brief Description of Drawings

FIG. 1 is a diagram schematically illustrating the entire configuration of an endoscope system according to a first embodiment of the present invention.
FIG. 2 is a partial sectional view of the distal end of an endoscope illustrated in FIG. 1.
FIG. 3 is partial sectional view of an imaging unit according to the first embodiment of the present invention.
FIG. 4A is a plan view illustrating an end face of a laminated circuit board according to the first embodiment of the present invention.
FIG. 4B is a schematic view illustrating a wiring pattern formed on the laminated circuit board according to the first embodiment of the present invention.
FIG. 5 is a view on arrow B of FIG. 3.
FIG. 6 is a partial sectional view of an imaging unit according to a second embodiment of the present invention.
FIG. 7 is a partial sectional view of an imaging unit according to a third embodiment of the present invention.
FIG. 8 is a partial sectional view of an imaging unit according to a fourth embodiment of the present invention.
FIG. 9 is a partial sectional view of an imaging unit according to a fifth embodiment of the present invention.
FIG. 10 is a sectional view illustrating a laminated circuit board according to a sixth embodiment of the present invention.

### Description of Embodiments

In the following description, an endoscope apparatus provided with an imaging unit will be described as a mode for carrying out the present invention (hereinbelow, referred to as "embodiment"). The invention is not limited to the embodiment. In the drawings, identical parts are designated by identical reference signs. It is to be noted that the drawings are schematic drawings, and the relationship between the thickness and the width in each member and the ratio of each member are different from the actual relationship and ratio. The dimension and the ratio may be partially different from each other between the drawings.

### (First Embodiment)

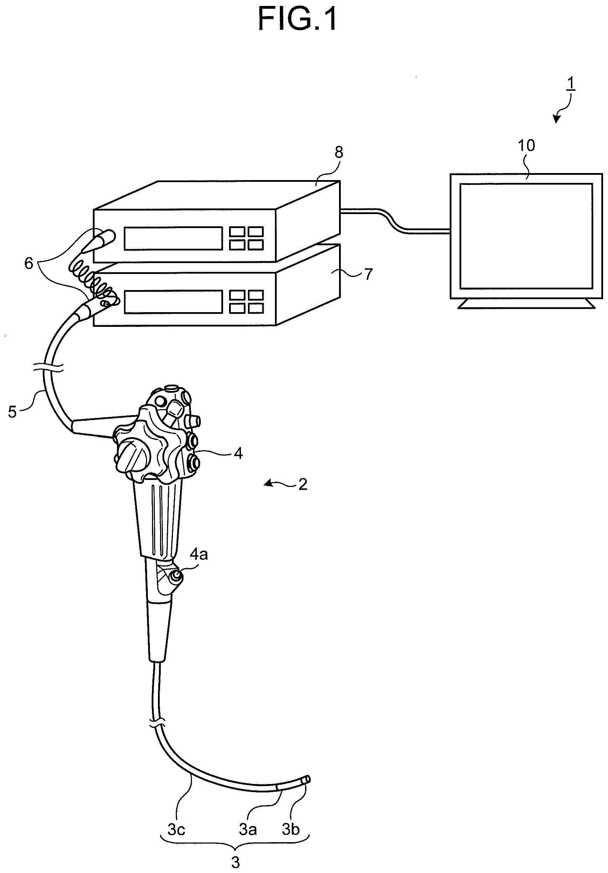

FIG. 1 is a diagram schematically illustrating the entire configuration of an endoscope system according to a first embodiment of the present invention. As illustrated in FIG. 1, an endoscope apparatus 1 is provided with an endoscope 2, a universal cord 5, a connector 6, a light source device 7, a processor (control device) 8, and a display device 10.

The endoscope 2 captures an in-vivo image of a subject and outputs an imaging signal by inserting an insertion part 3 into the body cavity of the subject. A cable inside the universal cord 5 is extended up to the distal end of the insertion part 3 of the endoscope 2 and connected to an imaging unit disposed on a distal end part 3b of the insertion part 3.

The connector 6 is disposed on the proximal end of the universal cord 5 and connected to the light source device 7 and the processor 8. The connector 6 applies predetermined signal processing to an imaging signal (output signal) output by the imaging unit disposed on the distal end part 3b which is connected to the universal cord 5, and analog-digital converts (A/D converts) the imaging signal and outputs the converted imaging signal as an image signal.

The light source device 7 is configured using, for example, a white LED. Pulse-like white light emitted by the light source device 7 forms illumination light that is applied to a subject from the distal end of the insertion part 3 of the endoscope 2 through the connector 6 and the universal cord 5.

The processor 8 applies predetermined image processing to an image signal output from the connector 6 and controls the entire endoscope apparatus 1. The display device 10 displays an image processed by the processor 8.

An operating unit 4 on which various kinds of buttons and knobs for operating an endoscope function are disposed is connected to the proximal end side of the insertion part 3 of the endoscope 2. The operating unit 4 is provided with a treatment tool insertion port 4a through which a treatment tool such as a biopsy forceps, an electrosurgical knife, and an inspection probe is inserted into the body cavity of a subject.

The insertion part 3 includes the distal end part 3b on which the imaging unit is disposed, a bendable part 3a which is formed continuously with the proximal end side of the distal end part 3b and freely bendable in upward and downward directions, and a flexible tube part 3c which is formed continuously with the proximal end side of the bendable part 3a. The bendable part 3a is bent in the upward and downward directions by an operation of a bending operation knob disposed on the operating unit 4 and freely bendable, for example, in two directions, specifically, the upward and downward directions, in response to pulling and relaxing of bending wires inserted through the inside of the insertion part 3. The upward and downward directions correspond to upward and downward directions in an image displayed on the display device 10. The upward and downward directions in the present specification are perpendicular to an extending direction (longitudinal direction) of the insertion part 3 and opposite to each other.

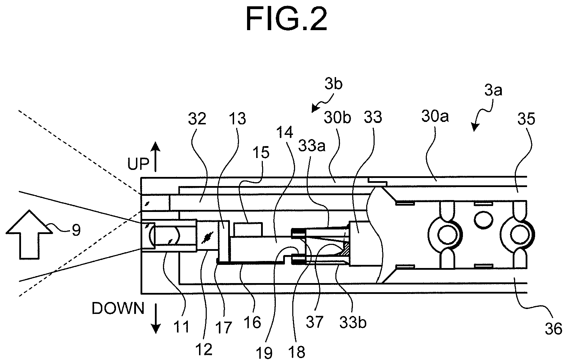

A light guide 32 (FIG. 2) which transmits illumination light from the light source device 7 is disposed on the endoscope 2, and a lens unit 11 (FIG. 2) is arranged on an outgoing end of illumination light by the light guide 32. The lens unit 11 is disposed on the distal end part 3b of the insertion part 3 and applies illumination light to a subject.

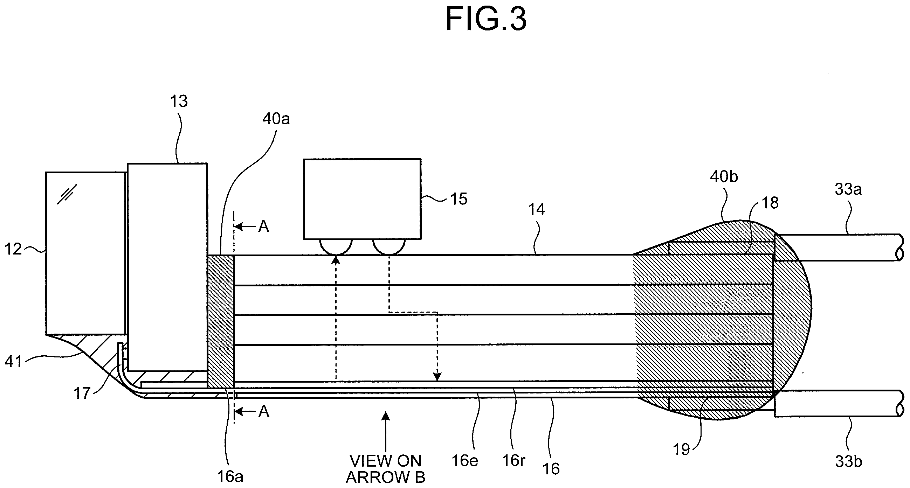

Next, the configuration of the distal end part 3b of the endoscope 2 will be described in detail. FIG. 2 is a partial sectional view of the distal end of the endoscope 2. FIG. 3 is a partial sectional view of the imaging unit according to the first embodiment of the present invention.

FIGS. 2 and 3 are sectional views cut on a plane that is perpendicular to a board surface of the imaging unit disposed on the distal end part 3b of the endoscope 2 and parallel to an optical axis direction of the imaging unit. FIG. 2 illustrates the distal end part 3b and a part of the bendable part 3a of the insertion part 3 of the endoscope 2. In FIG. 2, the upward direction (UP) corresponds to an bending upward direction of the bendable part 3a and the upward direction in an image displayed on the display device 10, and the downward direction (DOWN) corresponds to a bending downward direction of the bendable part 3a and the downward direction in an image displayed on the display device 10.

As illustrated in FIG. 2, the bendable part 3a is freely bendable in the upward and downward directions in response to pulling and relaxing of an upward-bending wire 35 and a downward-bending wire 36 which are inserted through a bending tube arranged inside a coated tube 30a. The imaging unit is disposed inside the distal end part 3b which extends from the distal end side of the bendable part 3a. The coated tube 30a is composed of a flexible member so that the bendable part 3a can be bent.

The imaging unit includes the lens unit 11 and a solid-state imaging element 13 which is arranged on the proximal end side of the lens unit 11. The imaging unit is adhered to the inside of a distal end part main body 30b with an adhesive. The distal end part main body 30b is formed of a hard member for forming an internal space which houses the imaging unit. The distal end part 3b in which the distal end part main body 30b is arranged constitutes a hard part of the insertion part 3. The length of the hard part (hard length) is defined between the distal end of the insertion part 3 and the proximal end of the distal end part main body 30b.

The lens unit 11 includes a plurality of objective lenses and a lens holder which holds the objective lenses. The lens unit 11 is fixed to the distal end part main body 30b by insert-fitting and fixing the distal end of the lens holder to the inside of the distal end part main body 30b.

The imaging unit is provided with the solid-state imaging element 13, such as a CCD and a CMOS, which generates an electric signal corresponding to incident light, a flexible circuit board 16 which extends in the optical axis direction from the solid-state imaging element 13, a laminated circuit board (hard circuit board) 14 which is formed on the surface of the flexible circuit board 16 and has a plurality of conductor layers, and a glass lid 12 which is adhered to the solid-state imaging element 13 to cover a light receiving surface on the surface of the solid-state imaging element 13.

An image of a subject 9 formed by the lens unit 11 is detected by the solid-state imaging element 13 which is disposed at an image formation position of the lens unit 11 and converted to an imaging signal. The imaging signal (output signal) is output to the processor 8 through the flexible circuit board 16, the laminated circuit board 14, an electronic component (second chip) 15, and a composite cable 33 (including a cable 33a and a cable 33b).

An inner lead 17 of the flexible circuit board 16 is electrically connected to a lower electrode of the solid-state imaging element 13 and the connected part is coated with a sealing resin (adhesive) 41. Accordingly, the solid-state imaging element 13 and the flexible circuit board 16 are connected to each other.

The flexible circuit board 16 is a flexible printed circuit board and extends in the optical axis direction of the solid-state imaging element 13 from the solid-state imaging element 13. The laminated circuit board 14 having a plurality of laminated layers is formed on the surface of the flexible circuit board 16 and electrically and mechanically connected to the flexible circuit board 16. In the first embodiment, a lamination direction of the laminated circuit board 14 is a direction perpendicular to the longitudinal direction of the insertion part 3 of the endoscope 2 (the direction parallel to the light receiving surface of the solid-state imaging element 13). Alternatively, the lamination direction of the laminated circuit board 14 may be the longitudinal direction of the insertion part 3 of the endoscope 2 (the direction perpendicular to the light receiving surface of the solid-state imaging element 13).

In the laminated circuit board 14 of the imaging unit, the electronic component 15 which constitutes, for example, a transmission buffer (second chip) for the solid-state imaging element 13 is mounted, and a via which allows the plurality of conductor layers to be electrically connected to each other is formed. The distal end of the cable 33a and the distal end of the cable 33b are connected to the proximal end of the laminated circuit board 14. An electronic component other than an electronic component that constitutes a driving circuit for the solid-state imaging element 13 may be mounted on the laminated circuit board 14.

A cable connection land 18 to which a conductor on the distal end of the cable 33a is electrically and mechanically connected is formed on the upper face of the laminated circuit board 14. A cable connection land 19 to which a conductor on the distal end of the cable 33b is electrically and mechanically connected is formed on the lower face of the laminated circuit board 14. A cable sheath of the cable 33a and a cable sheath of the cable 33b are disposed on the rear side with respect to the proximal end of the laminated circuit board 14. Thus, the cable sheaths do not overlap the laminated circuit board 14. The cable 33a and the cable 33b are cables including at least one selected from a signal line for a driving signal, a signal line for a power source, and a signal line for an output signal for the solid-state imaging element 13. Further, a dummy cable for heat dissipation may be included. Shield wires 37 of the composite cable 33 and the cable 33a and the cable 33b which constitute the composite cable 33 are collected together and connected to a grounding land formed on the lower face of the laminated circuit board 14.

One or more of a plurality of electronic components which constitute the driving circuit for the solid-state imaging element 13 are mounted on the surface of the upper part of the laminated circuit board 14 and one or more of the electronic components are also embedded and thereby mounted inside thereof. The entire imaging unit including the laminated circuit board 14, the electronic component 15, the flexible circuit board 16, and the composite cable 33 is arranged in such a manner that the entire imaging unit is located within a projected region formed by projecting the solid-state imaging element 13 in the optical axis direction.

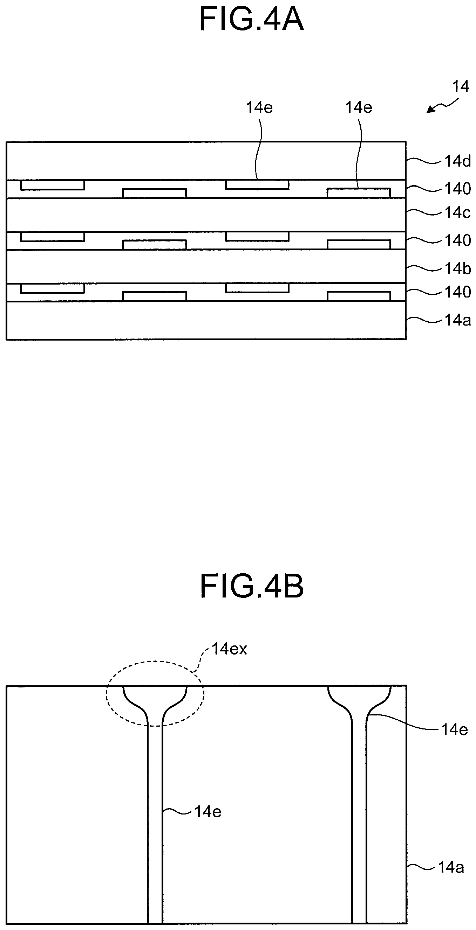

As illustrated in FIG. 3, a highly thermal conductive member (first heat dissipation member) 40a having a thermal conductivity of a predetermined value or more, for example, 0.2 mW/m/K or more is arranged between the rear face of the solid-state imaging element 13 and a side face of the laminated circuit board 14, the side face facing the solid-state imaging element 13. As described below with reference to FIG. 4A, a wiring pattern 14e is exposed on the side face of the laminated circuit board 14, the side face facing the solid-state imaging element 13. The wiring pattern 14e is electrically connected to the cable 33a or/and the cable 33b.

Heat generated by driving the solid-state imaging element 13 is transmitted from the rear face of the solid-state imaging element 13 to the wiring pattern 14e of the laminated circuit board 14 through the highly thermal conductive member 40a. The highly thermal conductive member 40a is, for example, an adhesive or a ceramic member having a high thermal conductivity and preferably has insulation properties. The wiring pattern 14e of the laminated circuit board 14 is electrically connected to the composite cable 33 through the connection lands and the like. Heat of the solid-state imaging element 13 is transmitted to the composite cable 33 through the highly thermal conductive member 40a and the wiring pattern 14e so as to be dissipated.

An opening 16a may be formed on a resist layer 16r on the flexible circuit board 16 to expose a wiring pattern 16e of the flexible circuit board 16 in a part having contact with the highly thermal conductive member 40a, and heat may be transmitted from the highly thermal conductive member 40a to the exposed wiring pattern 16e. Heat of the solid-state imaging element 13 that has been transmitted from the highly thermal conductive member 40a to the exposed wiring pattern 16e passes through the wiring pattern 16e, and is transmitted to the composite cable 33 through the cable connection lands 18, 19 and the like so as to be dissipated.

The proximal end part of the laminated circuit board 14 including a part connected to the cable 33a and the cable 33b may be coated with an insulative and highly thermal conductive adhesive (second heat dissipation member) 40b having a thermal conductivity of a predetermined value or more, for example, 0.2 mW/m/K or more. In such a configuration, heat that has been transmitted from the solid-state imaging element 13 to the laminated circuit board 14 through the highly thermal conductive member 40a is transmitted to all the cable 33a and the cable 33b included in the composite cable 33. Since the cable 33a and the cable 33b included in the composite cable 33 are connected to each other with the adhesive 40b, even when, for example, heat is transmitted only to the cable 33a through the wiring pattern 14e, the heart can be transmitted also to the cable 33b through the adhesive 40b. The wiring pattern 14e of the laminated circuit board 14 may be exposed also on the proximal end part of the laminated circuit board 14 in the same manner as in the distal end part thereof.

FIG. 4A is a plan view illustrating an end face of the laminated circuit board according to the first embodiment of the present invention. The end face of the laminated circuit board 14 illustrated in FIG. 4A is located on the distal end side (the side facing the solid-state imaging element 13) of the laminated circuit board 14. FIG. 4A is a sectional view of the distal end of the endoscope illustrated in FIG. 3 taken along line A-A.

The laminated circuit board 14 is formed by adhering a conductor layers 14a to 14d each having the wiring pattern 14e formed on the surface (the upper face or the lower face) thereof with an adhesive 140. As illustrated in FIG. 4A, the side face of the wiring pattern 14e is exposed on the end face on the distal end side (the side facing the solid-state imaging element 13) of the laminated circuit board 14. Exposing the end face of the wiring pattern 14e in this manner enables the side face to have direct contact with the highly thermal conductive member 40a. Accordingly, heat that has been transmitted from the solid-state imaging element 13 to the highly thermal conductive member 40a can be efficiently transmitted to the wiring pattern 14e.

The wiring pattern 14e exposed on the end face on the distal end side (the side facing the solid-state imaging element 13) of the laminated circuit board 14 may be a pattern for any signal, but preferably a solid pattern for ground having a large pattern area. When the wiring pattern 14e is a wiring pattern for ground or power source, the highly thermal conductive member 40a may be an electrically conductive member.

FIG. 4B is a schematic view illustrating the wiring pattern formed on the laminated circuit board according to the first embodiment of the present invention. FIG. 4B illustrates the upper face of the conductor layer 14a of the laminated circuit board 14. The wiring pattern 14e is formed on the upper face of the conductor layer 14a. The width of the wiring pattern 14e on an end 14ex thereof near the end of the conductor layer 14a is larger than the width of the wiring pattern 14e on a central part of the conductor layer 14a. Accordingly, the width of the wiring pattern 14e exposed on the end face of the laminated circuit board 14 becomes larger, which enables the exposed area of the wiring pattern 14e to be increased. Thus, the heat dissipation efficiency can be improved. The width of the wiring pattern 14e may be uniform.

Although FIGS. 4A and 4B illustrate the end face on the distal end side (the side facing the solid-state imaging element 13) of the laminated circuit board 14, an end face on the proximal end side (the side facing the composite cable 33) of the laminated circuit board 14 may have the same structure. That is, an end face of the wiring pattern 14e may be exposed on the end face on the proximal end side of the laminated circuit board 14. Along with this, the width of the end 14ex may be increased to increase the exposed area of the end face.

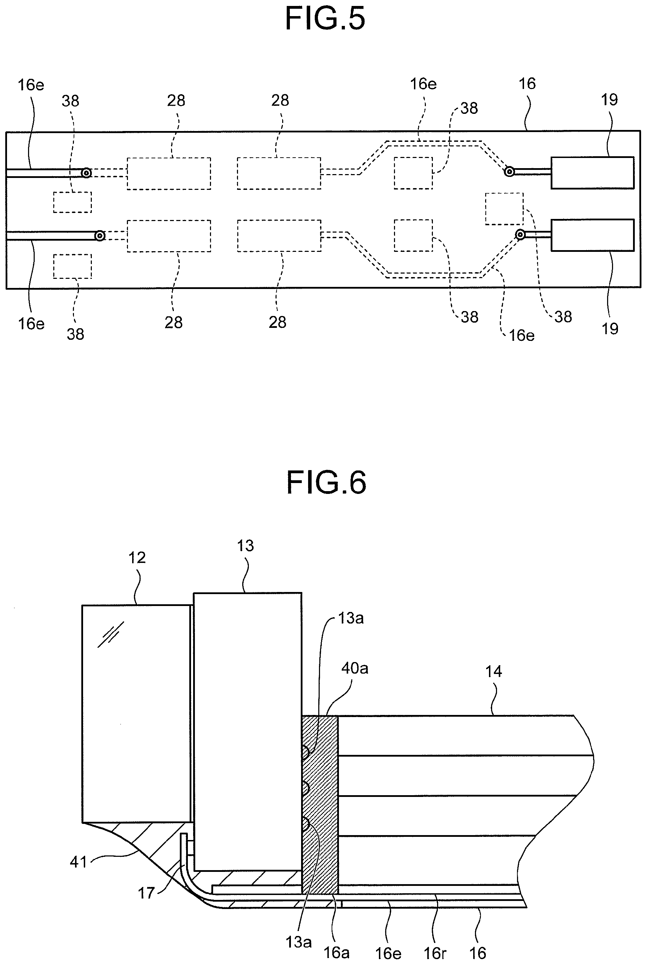

FIG. 5 is a view on arrow B of FIG. 3. FIG. 5 illustrates the lower face (the face that is not connected to the laminated circuit board 14) of the flexible circuit board 16. In the drawing, members indicated by dotted lines are disposed on the upper face (the face that is connected to the laminated circuit board 14) of the flexible circuit board 16.

The cable connection land 19 is disposed on the proximal end side of the lower face of the flexible circuit board 16 for connection with the composite cable 33. A board connection land 28 is disposed on the upper face of the flexible circuit board 16 for connection with the laminated circuit board 14. The board connection land 28 and the cable connection land 19 are electrically connected to each other through the wiring pattern 16e.

In a conventional structure, the flexible circuit board 16 and the laminated circuit board 14 are adhered with each other, for example, with an adhesive in the board connection land 28. Since the board connection land 28 and the cable connection land 19 are electrically connected to each other through the wiring pattern 16e, solder heat generated when the composite cable 33 is connected to the cable connection land 19 may be transmitted from the cable connection land 19 to the board connection land 28 through the wiring pattern 16e to melt the adhesive and the flexible circuit board 16 may thereby be separated from the laminated circuit board 14 and warped.

In the present embodiment, a connection reinforcement land 38 which is not connected to the cable connection land 19 is disposed on the upper face of the flexible circuit board 16 to adhere the flexible circuit board 16 and the laminated circuit board 14 with each other, for example, with an adhesive also in the connection reinforcement land 38. Since the connection reinforcement land 38 is not connected to the cable connection land 19, solder heat generated when the composite cable 33 is connected to the cable connection land 19 is not transmitted to the connection reinforcement land 38. Thus, remelting of the adhesive can be prevented. Accordingly, it is possible to prevent the flexible circuit board 16 from being separated from the laminated circuit board 14 and warped.

As described above, the first embodiment of the present invention makes it possible to transmit heat generated during driving of the solid-state imaging element 13 to the wiring pattern 14e exposed on the end face on the distal end side (the side facing the solid-state imaging element 13) of the laminated circuit board 14 through the highly thermal conductive member 40a disposed in contact with the rear face of the solid-state imaging element 13. Accordingly, heat of the solid-state imaging element 13 is transmitted to the composite cable 33 through the wiring pattern 14e and efficiently dissipated.

### (Second Embodiment)

FIG. 6 is a partial sectional view of an imaging unit according to a second embodiment of the present invention. In the second embodiment, in addition to the first embodiment, an electrode 13a for heat dissipation is disposed on the rear face of the solid-state imaging element 13. The electrode 13a for heat dissipation includes, for example, a BGA. The electrode 13a for heat dissipation may include a metal layer. The electrode 13a for heat dissipation disposed on the rear face of the solid-state imaging element 13 enables heat of the solid-state imaging element 13 to be more efficiently transmitted to the highly thermal conductive member 40a.

### (Third Embodiment)

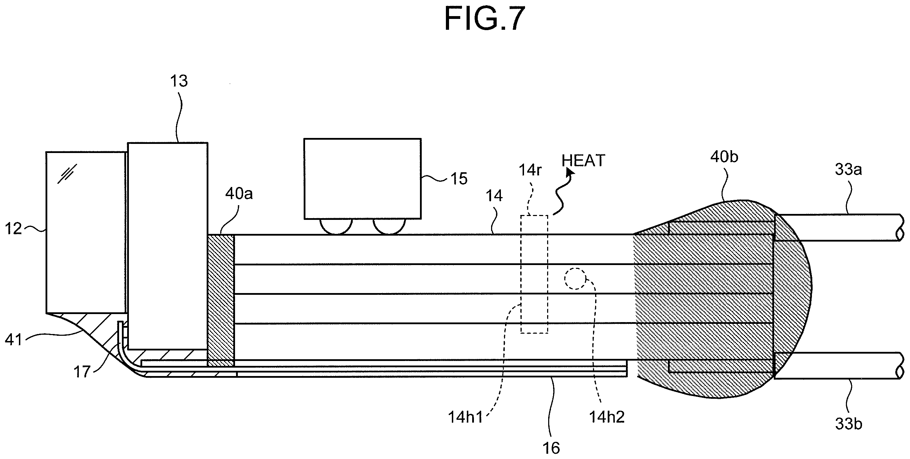

FIG. 7 is a partial sectional view of an imaging unit according to a third embodiment of the present invention. In the third embodiment, in addition to the first embodiment, a hole 14h1 is formed in the lamination direction (vertical direction) in a part of the laminated circuit board 14 in which the wiring pattern 14e is not dense and a heat dissipation member 14r made of metal is inserted into the hole 14h1 to thereby dissipate heat transmitted from the solid-state imaging element 13 to the laminated circuit board 14. Further, in addition to the hole 14h1 or instead of the hole 14h1, a hole 14h2 may be formed not in the lamination direction, but in a direction perpendicular to the lamination direction (horizontal direction). Further, another metal member such as a heat dissipation wire may be connected to the heat dissipation member 14r to improve the heat dissipation efficiency.

### (Fourth Embodiment)

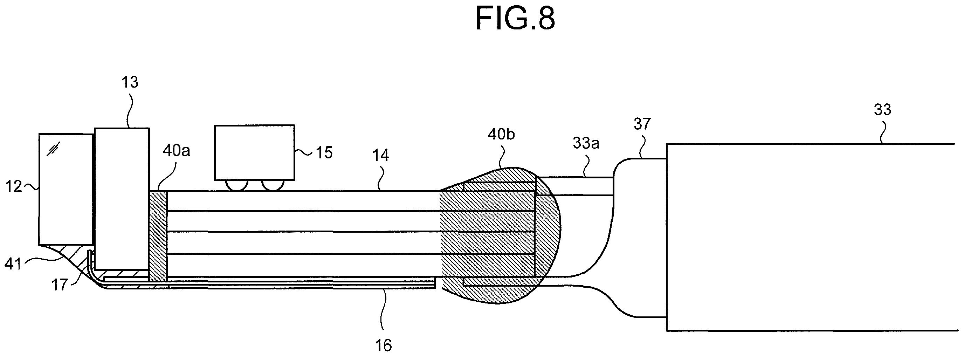

FIG. 8 is a partial sectional view of an imaging unit according to a fourth embodiment of the present invention. In the fourth embodiment, in addition to the first embodiment, a shield wire 37 of the composite cable 33 is connected to the laminated circuit board 14 and the connected part is coated with a highly thermal conductive adhesive 40b to thereby transmit heat that has been transmitted from the solid-state imaging element 13 to the laminated circuit board 14 to the shield wire 37 of the composite cable 33 to improve the heat dissipation efficiency.

### (Fifth Embodiment)

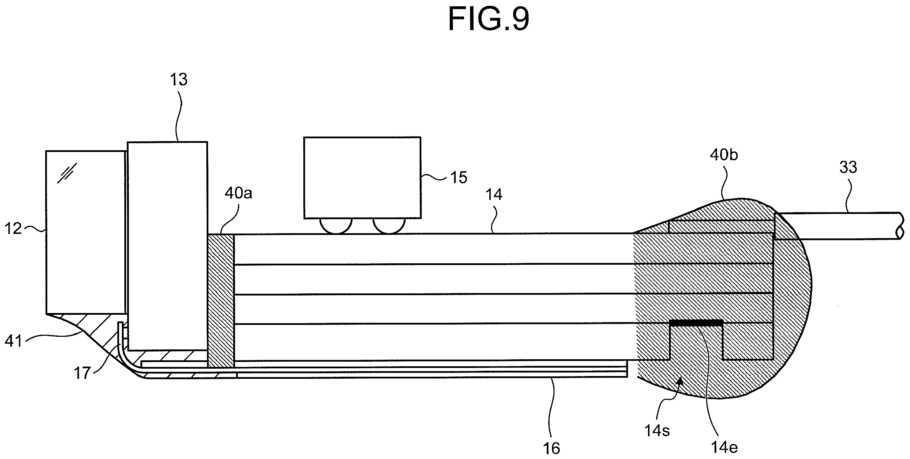

FIG. 9 is a partial sectional view of an imaging unit according to a fifth embodiment of the present invention. In the fifth embodiment, in addition to the first embodiment, a cut-away part 14s is formed on the laminated circuit board 14. The cut-away part 14s is preferably formed in a region coated with an adhesive 40b on the proximal end part side of the laminated circuit board 14 as illustrated in FIG. 9, but may be formed in another place. Inside the cut-away part 14s, the end faces and the upper face of the wiring pattern 14e are exposed. The cut-away part 14s enables the exposed area of the wiring pattern 14e to be increased. Accordingly, the heat dissipation efficiency can be improved.

### (Sixth Embodiment)

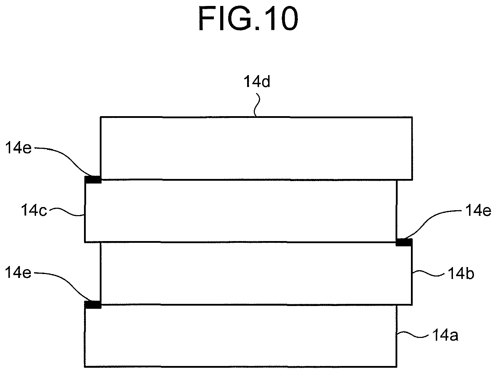

FIG. 10 is a sectional view illustrating a laminated circuit board according to a sixth embodiment of the present invention. In the sixth embodiment, conductor layers 14a to 14d of a laminated circuit board 14 are laminated in such a manner that the conductor layers 14a to 14d are alternately displaced in a direction perpendicular to the lamination direction to expose the upper face of the wiring pattern 14e on the end faces of the laminated circuit board 14. The conductor layers 14a to 14d may be displaced in any of a front-rear direction and a right-left direction when a direction corresponding to the distal end and the proximal end of the laminated circuit board 14 is defined as the front-rear direction. Further, the conductor layers 14a to 14d may be displaced in the front-rear direction only on either the distal end part or the proximal end part of the laminated circuit board 14. Alternately displacing the conductor layers 14a to 14d enables the upper face of the wiring pattern 14e to be exposed to increase the exposed area of the wiring pattern 14e. Accordingly, the heat dissipation efficiency can be improved.

### Reference Signs List

1 endoscope apparatus
2 endoscope
3a bendable part
3b distal end part
3c flexible tube part
4 operating unit
4a treatment tool insertion port
5 universal cord
6 connector
7 light source device
8 processor
9 subject
10 display device
11 lens unit
12 glass lid
13 solid-state imaging element
14 laminated circuit board
14a, 14b, 14c, 14d conductor layer
14e, 16e wiring pattern
14h1, 14h2 hole
14r heat dissipation member
14s cut-away part
15 electronic component
16 flexible circuit board
16a opening
16r resist layer
17 inner lead
18, 19 cable connection land
28 board connection land
30a coated tube
30b distal end part main body
32 light guide
33 composite cable
33a, 33b cable
35 upward-bending wire
36 downward-bending wire
37 shield wire
38 connection reinforcement land
40a highly thermal conductive member (first heat dissipation member)
40b adhesive (second heat dissipation member)
41 sealing resin

## Claims

1. An endoscope apparatus (1) comprising:
a solid-state imaging element (13) including a light receiving surface on a front face thereof;
a circuit board (16) arranged on a rear face side of the solid-state imaging element (13);
a laminated circuit board (14) formed on an upper surface of the circuit board (16) and having a plurality of conductor layers, the laminated circuit board (14) including a wiring pattern (14e) a part of which is exposed on a distal end side of the circuit board (16), a distal end side of the laminated circuit board (14) facing the solid-state imaging element (13);
a first heat dissipation member (40a) arranged between the solid-state imaging element (13) and the exposed part of the wiring pattern (14e), the first heat dissipation member (40a) being in contact with the rear face of the solid-state imaging element (13) and the exposed part of the wiring pattern (14e);
a cable (33a, 33b) electrically connected to the wiring pattern (14e);
a cable connection land (19) disposed on a proximal end side of a lower surface of the circuit board (16) for connection with the cable (33b);
a board connection land (28) disposed on the upper face of the circuit board (16) for connection with the laminated circuit board (14),
wherein the circuit board (16) comprises an opening (16a) formed on a resist layer (16r) on the circuit board (16) to expose a wiring pattern (16e) having contact with the first heat dissipation member (40a);
wherein a width (14ex) of the exposed part of the wiring pattern (14e) in contact with the first heat dissipation member (40a) is wider than a width of the wiring pattern (14e) at a central part of the laminated circuit board (14);
wherein the board connection land (28) and the cable connection land (19) are electrically connected to each other through the wiring pattern (16e), and
wherein the circuit board (16) and the laminated circuit board (14) are adhered with each other with an adhesive in the board connection land (28).

2. The endoscope apparatus according to claim 1, wherein
the circuit board (16) comprises:
the laminated circuit board (14) including the plurality of conductor layers laminated in a direction parallel to the light receiving surface; and
a soft circuit board (17) configured to connect the solid-state imaging element (13) electrically to the laminated circuit board (14), and
the part of the wiring pattern (14e) is exposed on an end face of the laminated circuit board (14), the end face facing the rear face of the solid-state imaging element (13).

3. The endoscope apparatus according to claim 1 or 2, further comprising an insulative second heat dissipation member (40b) configured to cover the cable (33) on a proximal end side of the circuit board (16).

4. The endoscope apparatus according to claim 3, wherein a part of a wiring pattern electrically connected to the wiring pattern (14e) exposed on the distal end side is exposed on the proximal end side of the circuit board (16).

5. The endoscope apparatus according to any one of claims 2 to 4, wherein the first heat dissipation member (40a) is an adhesive that fixes the solid-state imaging element (13) and the laminated circuit board (14) to each other.

6. The endoscope apparatus according to any one of claims 1 to 5, wherein the first heat dissipation member (40a) is an insulative member.

7. The endoscope apparatus according to any one of claims 1 to 5, wherein the first heat dissipation member (40a) is an electrically conductive member, and the wiring pattern (14e) is a pattern for ground or power source.

## Patentansprüche

1. Endoskopvorrichtung (1) umfassend:
ein Festkörperbildgebungselement (13), welches eine Lichtempfangsoberfläche auf eine Vorderseite enthält;
eine Leiterplatte (16), welche auf einer Rückseite des Festkörperbildgebungselements (13) angeordnet ist;
eine beschichtete Leiterplatte (14), welche auf einer oberen Seite der Leiterplatte (16) gebildet ist und eine Vielzahl von Leiterschichten aufweist, wobei die beschichtete Leiterplatte (14) ein Verkabelungsmuster (14e) enthält, wovon ein Teil auf einer distalen Endseite der Leiterplatte (16) freigelegt ist, und eine distale Endseite der beschichteten Leiterplatte (14) dem Festkörperbildgebungselement (13) zugewandt ist;
ein erstes Wärmeabführteil (40a), welches zwischen dem Festkörperbildgebungselement (13) und dem freigelegten Teil des Verkabelungsmusters (14e) angeordnet ist, wobei das erste Wärmeabführteil (40a) in Kontakt mit der Rückseite des Festkörperbildgebungselements (13) und dem freigelegten Teil des Verkabelungsmusters (14e) steht;
ein Kabel (33a, 33b), welches elektrisch mit dem Verkabelungsmuster (14e) verbunden ist;
eine Kabelanschlussfläche (19), welche zum Verbinden mit dem Kabel (33b) auf einer proximalen Endseite der Unterseite der Leiterplatte (16) angeordnet ist;
eine Plattenanschlussfläche (28), welche zum Verbinden mit der beschichteten Leiterplatte (14) auf der oberen Seite der Leiterplatte (16) angeordnet ist,
wobei die Leiterplatte (16) eine Öffnung (16a) umfasst, welche auf einer Resistschicht (16r) auf der Leiterplatte (16) gebildet ist, um ein Verkabelungsmuster (16e) freizulegen, welches mit dem ersten Wärmeabführteil (40a) in Kontakt steht;
wobei eine Breite (14ex) des freigelegten Teils des Verkabelungsmusters (14e), welches mit dem ersten Wärmeabführteil (40a) in Kontakt steht, größer ist als eine Breite des Verkabelungsmusters (14e) an einem zentralen Teil der beschichteten Leiterplatte (14);
wobei die Plattenanschlussfläche (28) und die Kabelanschlussfläche (19) durch das Verkabelungsmuster (16e) elektrisch miteinander verbunden sind, und
wobei die Leiterplatte (16) und die beschichtete Leiterplatte (14) mit einem Kleber an der Plattenanschlussfläche (28) miteinander verklebt sind.

2. Endoskopvorrichtung gemäß Anspruch 1, wobei
die Leiterplatte (16) umfasst:
die beschichtete Leiterplatte (14), welche die Vielzahl von Leiterschichten enthält, welche in einer Richtung parallel zu der Lichtempfangsoberfläche beschichtet sind; und
eine weiche Leiterplatte (17), welche konfiguriert ist, um das Festkörperbildgebungselement (13) mit der beschichteten Leiterplatte (14) elektrisch zu verbinden, und
der Teil des Verkabelungsmusters (14e) auf einer Endseite der beschichteten Leiterplatte (14) freigelegt ist, wobei die Endseite der Rückseite des Festkörperbildgebungselements (13) zugewandt ist.

3. Endoskopvorrichtung gemäß Anspruch 1 oder 2, des Weiteren ein isolierendes zweites Wärmeabführteil (40b) umfassend, welches konfiguriert ist, um das Kabel (33) an einer proximalen Endseite der Leiterplatte (16) zu bedecken.

4. Endoskopvorrichtung gemäß Anspruch 3, wobei ein Teil eines Verkabelungsmusters, welches mit dem Verkabelungsmuster (14e) elektrisch verbunden ist, welches an dem distalen Ende freigelegt ist, an der proximalen Endseite der Leiterplatte (16) freigelegt ist.

5. Endoskopvorrichtung gemäß einem der Ansprüche 2 bis 4, wobei das erste Wärmeabführteil (40a) ein Kleber ist, welcher das Festkörperbildgebungselement (13) und die beschichtete Leiterplatte (14) aneinander befestigt.

6. Endoskopvorrichtung gemäß einem der Ansprüche 1 bis 5, wobei das erste Wärmeabführteil (40a) ein isolierendes Teil ist.

7. Endoskopvorrichtung gemäß einem der Ansprüche 1 bis 5, wobei das erste Wärmeabführteil (40a) ein elektrisch leitendes Teil ist, und das Verkabelungsmuster (14e) ein Muster zur Erdung oder als Energiequelle ist.

## Revendications

1. Appareil d'endoscope (1) comprenant :
un élément d'imagerie à semi-conducteur (13) incluant une surface de réception de lumière sur une face avant de celui-ci ;
une carte de circuit (16) agencée sur un côté de face arrière de l'élément d'imagerie à semi-conducteur (13) ;
une carte de circuit stratifiée (14) formée sur une surface supérieure de la carte de circuit (16) et comportant une pluralité de couches conductrices, la carte de circuit stratifiée (14) incluant un motif de câblage (14e) dont une partie est exposée sur un côté d'extrémité distale de la carte de circuit (16), un côté d'extrémité distale de la carte de circuit stratifiée (14) étant en regard de l'élément d'imagerie à semi-conducteur (13) ;
un premier élément de dissipation de chaleur (40a) agencé entre l'élément d'imagerie à semi-conducteur (13) et la partie exposée du motif de câblage (14e), le premier élément de dissipation de chaleur (40a) étant en contact avec la face arrière de l'élément d'imagerie à semi-conducteur (13) et la partie exposée du motif de câblage (14e) ;
un câble (33a, 33b) électriquement connecté au motif de câblage (14e) ;
une plage de connexion de câble (19) disposée sur un côté d'extrémité proximale d'une surface inférieure de la carte de circuit (16) pour connexion avec le câble (33b) ;
une plage de connexion de carte (28) disposée sur la surface supérieure de la carte de circuit (16) pour connexion avec la carte de circuit stratifiée (14),
dans lequel la carte de circuit (16) comprend une ouverture (16a) formée sur une couche de réserve (16r) sur la carte de circuit (16) pour exposer un motif de câblage (16e) étant en contact avec le premier élément de dissipation de chaleur (40a) ;
dans lequel une largeur (14ex) de la partie exposée du motif de câblage (14e) en contact avec le premier élément de dissipation de chaleur (40a) est plus large qu'une largeur du motif de câblage (14e) au niveau d'une partie centrale de la carte de circuit stratifiée (14) ;
dans lequel la plage de connexion de carte (28) et la plage de connexion de câble (19) sont électriquement connectées l'une à l'autre par l'intermédiaire d'un motif de câblage (16e), et
dans lequel la carte de circuit (16) et la carte de circuit stratifiée (14) sont collées l'une à l'autre par un adhésif dans la plage de connexion de carte (28).

2. Appareil d'endoscope selon la revendication 1, dans lequel
la carte de circuit (16) comprend :
la carte de circuit stratifiée (14) incluant la pluralité de couches conductrices stratifiées dans une direction parallèle à la surface de réception de lumière ; et
une carte de circuit souple (17) configurée pour connecter électriquement l'élément d'imagerie à semi-conducteur (13) à la carte de circuit stratifiée (14), et
la partie du motif de câblage (14e) est exposée sur une face d'extrémité de la carte de circuit stratifiée (14), la face d'extrémité étant en regard de la face arrière de l'élément d'imagerie à semi-conducteur (13).

3. Appareil d'endoscope selon la revendication 1 ou 2, comprenant en outre un deuxième élément de dissipation de chaleur (40b) isolant configuré pour couvrir le câble (33) sur un côté d'extrémité proximale de la carte de circuit (16).

4. Appareil d'endoscope selon la revendication 3, dans lequel une partie d'un motif de câblage électriquement connectée au motif de câblage (14e) exposé sur le côté d'extrémité distale est exposée sur le côté d'extrémité proximale de la carte de circuit (16).

5. Appareil d'endoscope selon l'une quelconque des revendications 2 à 4, dans lequel le premier élément de dissipation de chaleur (40a) est un adhésif qui fixe l'élément d'imagerie à semi-conducteur (13) et la carte de circuit stratifiée (14) l'un à l'autre.

6. Appareil d'endoscope selon l'une quelconque des revendications 1 à 5, dans lequel le premier élément de dissipation de chaleur (40a) est un élément isolant.

7. Appareil d'endoscope selon l'une quelconque des revendications 1 à 5, dans lequel le premier élément de dissipation de chaleur (40a) est un élément électriquement conducteur, et le motif de câblage (14e) est un motif pour la masse ou la source d'alimentation.
